# EUROPEAN PATENT APPLICATION

(11) **EP 2 256 191 A1**
(43) Date of publication of application: **01.12.2010**
(21) Application number: 09710596.9
(22) Date of filing: 13.02.2009
(51) Int. Cl.: C12N 15/09, A61K 31/7105, A61K 47/34, A61K 47/36, A61K 47/48

(54) **CYCLIC SINGLE-STRANDED NUCLEIC ACID COMPLEX AND METHOD FOR PRODUCING THE SAME**

(30) Priority: 15.02.2008 JP 2008035310
(71) Applicant: Riken, Wako-shi, Saitama 351-0198 (JP)
(72) Inventor: ABE, Hiroshi, Wako-shi Saitama 351-0198 (JP); ITO, Yoshihiro, Wako-shi Saitama 351-0198 (JP); ABE, Naoko, Wako-shi Saitama 351-0198 (JP); HARADA, Mitsuru, Wako-shi Saitama 351-0198 (JP)
(74) Representative: Woods, Geoffrey Corlett
(86) International application number: PCT/JP2009/052945
(87) International publication number: WO 2009/102081

(57) **Abstract**

The present invention relates to a circular single-stranded nucleic acid complex comprising a sense strand RNA sequence, an antisense strand RNA sequence complementary to the sense strand RNA sequence, and two identical or different loop moieties connecting the sense and antisense strands, wherein the sense and antisense strands form a stem by pairing, and the loop moieties are selected from the group consisting of polyalkylene glycol, DNA, DNA-RNA chimera, a derivative thereof, and a combination thereof.

## Description

### Technical Field

The present invention relates to a circular single-stranded nucleic acid complex, a method for producing the same, and a pharmaceutical composition comprising the circular single-stranded nucleic acid complex.

### Background Art

The RNA interference method is broadly classified into a method using chemically synthesized double-stranded RNA and a method using a plasmid vector. Drug development using this RNA interference method is currently under way. Since the latter method using a plasmid vector has the problem of safety to human bodies, drug development using the former method using chemically synthesized double-stranded RNA has been expected.

However, double-stranded RNA is easily degraded by intracellular nuclease when administered to living bodies and thus had the problem of *in-vivo* instability.

For enhancing the *in-vivo* stability of double-stranded RNA, an RNA strand has been developed, which comprises unnatural nucleoside introduced therein. However, such an RNA strand had the problem of decreased bioactivity, though its stability is enhanced. Furthermore, the RNA strand was difficult to apply to drugs, because the toxicity of the unnatural nucleoside to living bodies is unknown.

In another approach of improving the *in-vivo* stability of double-stranded RNA, circular single-stranded RNA (dumbbell-shaped RNA) has been developed. The dumbbell-shaped RNA is free from RNA ends, because it is closed by loops at both the ends of the double-stranded RNA. Thus, this RNA is less likely to become a substrate for a degrading enzyme such as exonuclease other than specific enzymes (e.g., dicer) in the cells and is thus less degraded. Examples of such dumbbell-shaped RNA include dumbbell-shaped RNA described in Abe, N. et al. (Dumbbell-Shaped Nanocircular RNAs for RNA Interference. Journal of the American Chemical Society, 2007, 129, 15108-15109). Abe et al. discloses that the dumbbell-shaped RNA is more stable in a buffer supplemented with human serum than double-stranded RNA.

Moreover, JP Patent Publication (Kokai) No. 11-137260A (1999) discloses a dumbbell-shaped RNA-DNA chimeric nucleic acid as an anti-influenza virus agent. This dumbbell-shaped nucleic acid is used in an antisense method that directly targets the influenza virus gene. The dumbbell-shaped nucleic acid has a stem moiety comprising DNA paired with its complementary RNA and loop moieties comprising an oligonucleotide or ethylene glycol compound residue. The dumbbell-shaped nucleic acid, when entering cells, is thought to be degraded or cleaved by ribonuclease H to release the antisense DNA moiety.

### Summary of the Invention

An object of the present invention is to provide a circular single-stranded nucleic acid complex used in the RNA interference method, a method for producing the same, and a pharmaceutical composition comprising the circular single-stranded nucleic acid complex.

The present inventors have completed the present invention by finding that, for example, a circular single-stranded nucleic acid complex is improved in *in-vivo* stability, intracellularly exhibits RNA interference effect, and can be produced simply and efficiently, by selecting two loop moieties linking to the stem moiety of the circular single-stranded nucleic acid complex, from polyalkylene glycol, DNA, DNA-RNA chimera, a derivative thereof, and a combination thereof.

The features of the present invention are summarized as follows:
[1] A circular single-stranded nucleic acid complex comprising a sense strand RNA sequence, an antisense strand RNA sequence complementary to the sense strand RNA sequence, and two identical or different loop moieties connecting the sense and antisense strands, wherein the sense and antisense strands form a stem by pairing, and the loop moieties are selected from the group consisting of polyalkylene glycol, DNA, DNA-RNA chimera, a derivative thereof, and a combination thereof.
[2] The circular single-stranded nucleic acid complex according to [1], wherein the circular single-stranded nucleic acid complex is intracellularly converted to double-stranded RNA having RNA interference effect.
[3] The circular single-stranded nucleic acid complex according to [1] or [2], wherein the RNA sequence forming the stem has 80 to 100% identity to a target RNA sequence.
[4] The circular single-stranded nucleic acid complex according to any of [1] to [3], wherein the polyalkylene glycol is polyethylene glycol.
[5] The circular single-stranded nucleic acid complex according to any of [1] to [4], wherein the stem has a length of 19 to 31 nucleotides.
[6] A method for producing the circular single-stranded nucleic acid complex according to any of [1] to [5], comprising the following steps:
   (1) preparing a linear single-stranded nucleic acid complex comprising a portion of a sense strand, a loop moiety, and a portion of an antisense strand, and a linear single-stranded nucleic acid complex comprising the remaining portion of the sense strand, a loop moiety, and the remaining portion of the antisense strand; and
   (2) linking the portion of the sense strand to the remaining portion of the sense strand and the portion of the antisense strand to the remaining portion of the antisense strand.
[7] A method for producing the circular single-stranded nucleic acid complex according to any of [1] to [5], comprising the following steps:
   (1) preparing sense and antisense strands; and
   (2) linking loop moieties to both the ends of double-stranded RNA formed by the sense and antisense strands.
[8] A method for inhibiting *in vitro* the expression of a gene encoding a protein, comprising introducing the circular single-stranded nucleic acid complex according to any of [1] to [5] into a eukaryotic cell to disable target RNA such that its translation into the protein is inhibited.
[9] A method for inhibiting the expression of a gene encoding a protein, comprising introducing the circular single-stranded nucleic acid complex according to any of [1] to [5] into a non-human animal, a plant, or a cell thereof to disable target RNA such that its translation into the protein is inhibited.
[10] A pharmaceutical composition comprising the circular single-stranded nucleic acid complex according to any of [1] to [5] as an active ingredient.

The "circular single-stranded nucleic acid complex" is also referred to as a "dumbbell-shaped nucleic acid complex" herein. The "dumbbell-shaped nucleic acid complex" refers to a circular single-stranded nucleic acid complex that is dumbbell-shaped as a whole in which the sense and antisense strands form a stem by complementary pairing, and loops are respectively formed at both the ends of the stem to link the sense and antisense strands. Moreover, the "nucleic acid complex" refers to a complex of DNA and RNA, a complex of polyalkylene glycol and RNA, or a complex of DNA, polyalkylene glycol, and RNA.

As used herein, the "DNA-RNA chimera" refers to a hybrid of DNA and RNA sequences in an arbitrary combination, and these sequences may be combined in any form. For example, a block of continuous DNAs may be linked to a block of continuous RNAs; a plurality of DNA blocks and a plurality of RNA blocks may be linked randomly; or DNAs and RNAs may be liked alternately.

As used herein, the "target RNA" refers to mRNA of a gene whose expression is to be inhibited by the RNA interference method, or a precursor RNA thereof.

The present specification encompasses the contents described in the specification and/or drawings of Japanese Patent Application No. 2008-035310 that serves as a basis of the priority of the present application.

### Brief Description of the Drawings

Figure 1 schematically shows a dumbbell-shaped nucleic acid complex of the present invention.
Figure 2 shows the summary of a method for producing the dumbbell-shaped nucleic acid complex of the present invention.
Figure 3 shows the summary of a method for producing the dumbbell-shaped nucleic acid complex of the present invention.
Figure 4 shows electrophoretic profiles of the dumbbell-shaped nucleic acid complex of the present invention, etc.
Figure 5 shows the structures of the dumbbell-shaped nucleic acid complex of the present invention and double-stranded RNA.
Figure 6 shows electrophoretic profiles of the dumbbell-shaped nucleic acid complex of the present invention, dumbbell-shaped RNA, and double-stranded RNA in a biological stability test with serum.
Figure 7 shows time-dependent change (survival rate) in quantification results based on bands in the electrophoretic profiles of the dumbbell-shaped nucleic acid complex of the present invention, dumbbell-shaped RNA, and double-stranded RNA in the biological stability test with serum.
Figure 8 shows electrophoretic profiles of the dumbbell-shaped nucleic acid complex of the present invention, dumbbell-shaped RNA, and double-stranded RNA in a biological stability test with cell extracts.
Figure 9 shows time-dependent change (survival rate) in quantification results based on bands in the electrophoretic profiles of the dumbbell-shaped nucleic acid complex of the present invention, dumbbell-shaped RNA, and double-stranded RNA in the biological stability test with cell extracts.
Figure 10 shows the RNA interference effects of the dumbbell-shaped nucleic acid complex of the present invention and double-stranded RNA in cultured mammalian cells.

### Description of the Preferred Embodiments

### 1. Circular single-stranded nucleic acid complex

A circular single-stranded nucleic acid complex (hereinafter, also referred to as a "dumbbell-shaped nucleic acid complex") of the present invention can provide RNA interference effect. The RNA interference effect is effect of degrading target RNA or inhibiting its translation intracellularly or *in vivo*, through the binding thereto of a small RNA molecule having a sequence complementary to the target RNA.

The dumbbell-shaped nucleic acid complex of the present invention is a circular single-stranded nucleic acid complex comprising a sense strand RNA sequence, an antisense strand RNA sequence substantially complementary to the sense strand RNA sequence, and two identical or different loop moieties connecting the sense and antisense strands.

The antisense strand RNA sequence is a sequence that is substantially complementary to the sense strand RNA sequence and specifically binds to target RNA in RNA interference. The sense and antisense strands form a stem by pairing. Specifically, the sense strand RNA sequence and the antisense strand RNA sequence have a target RNA sequence and a sequence complementary thereto, respectively, or an RNA sequence having 80 to 100% identity to a target RNA sequence and a sequence complementary thereto, respectively. In this context, the "identity" represents the ratio (%) of the number of identical nucleotides to the total number of nucleotides between two nucleotide sequences aligned with or without an introduced gap. The length of the stem is not particularly limited as long as it imparts RNA interference effect. The length is, for example, 19 to 31 base pairs, preferably 20 to 25 base pairs, more preferably 20 to 24 base pairs, even more preferably 20 to 23 base pairs.

The RNAs of the sense and/or antisense strands may be a derivative comprising modified nucleoside. The modified nucleoside is a modified form of the base and sugar moieties, and examples thereof include alkylated forms, alkoxylated forms, inosine, 2'-O-alkylated or 2'-O-halogenated forms of ribose, and 2',4'-BNA forms. Moreover, the phosphate group moiety of the nucleotide may be modified, and examples of the modified form of the phosphate group moiety include phosphorothioate-modified nucleotide and methylphosphonate. Moreover, the nucleotide sequences of the sense and/or antisense strands may be not only RNA but also RNA-DNA chimera as long as they have RNA interference effect. Furthermore, the sense strand may contain 1- to 6-base mismatch, preferably 1- to 3-base mismatch, more preferably 1- or 2-base mismatch. The sense strand containing base mismatch is thought to allow double-stranded RNA to easily dissociate into single-stranded RNAs by helicase (Schwarz, D.S., et al. Asymmetry in the Assembly of the RNAi Enzyme Complex. Cell 115, 199-208 (2003)). The loop moieties are respectively located at both the ends of the stem. They link the 5'-terminal RNA of the sense strand to the 3'-teminal RNA of the antisense strand and link the 3'-terminal RNA of the sense strand to the 5'-terminal RNA of the antisense strand. The loop moieties are composed of polyalkylene glycol, DNA, DNA-RNA chimera, a derivative thereof, or a combination thereof. The resulting dumbbell-shaped nucleic acid complex is more improved in intracellular or *in-vivo* stability than, for example, double-stranded RNA (siRNA), circular single-stranded RNA comprising loop moieties consisting of only RNA, or double-stranded RNA having a hairpin structure (shRNA) and can thus exert more lasting or sustained-release effect in RNA interference. The lengths of the loops are not particularly limited and may be any length that can link the 5' and 3' ends on each side of the stem. Even if the loops are too long, RNA cleavage by dicer is not inhibited.

The polyalkylene glycol in the loop moieties is not particularly limited and is preferably highly safe to living bodies. The polyalkylene glycol encompasses derivatives in which the alkylene moiety may be substituted by a non-limiting substituent such as halogen, alkyl, hydroxy, alkoxy, acyl, or alkylthio. Moreover, carbon number of its monomer unit is preferably 1 to 4 and may be 5 or more, for example, 5 to 8. Examples of the polyalkylene glycol include polyethylene glycol, polypropylene glycol, polybutylene glycol, poly(oxyethylene-oxypropylene) glycol, poly(oxyethylene-oxybutylene) glycol, polyoxyethylene-polyoxypropylene glycol, and polyoxyethylene-polyoxybutylene glycol. In the present invention, polyethylene glycol is particularly preferable.

The polyethylene glycol is represented by the formula: -O-[(CH₂)₂-O-]ₙ-, wherein n is not particularly limited and is preferably n=1 to 1000, more preferably n=2 to 50, even more preferably 3 to 20, particularly preferably 3 to 10. Either or both of the hydroxyl groups of the polyalkylene glycol may be phosphoric acid-esterified.

The DNA or DNA-RNA chimera in the loop moieties may have a loop length of 1 nucleotide or more, preferably 2 to 20 nucleotides, more preferably 6 to 12 nucleotides, even more preferably 7 to 11 nucleotides. The nucleic acid sequences of the loop moieties are not particularly limited as long as they comprise a sequence that does not substantially form a complementary strand. Examples of a DNA sequence that does not form a complementary strand include GATTAGTCACT (SEQ ID NO: 14), AGAGAACTT (SEQ ID NO: 15), TTCAAGAGA (SEQ ID NO: 16), and TGTGCTGTC (SEQ ID NO: 17). These DNA sequences may be substituted partially by RNA corresponding to each DNA. The DNA or RNA may be a derivative comprising modified nucleoside. The modified nucleoside is a modified form of the base and sugar moieties, and examples thereof include alkylated forms, alkoxylated forms, inosine, 2'-O-alkylated or 2'-O-halogenated forms of ribose, and 2',4'-BNA forms. Moreover, the phosphate group moiety of the nucleotide may be modified, and examples of the modified form of the phosphate group moiety include phosphorothioate-modified nucleotide and methylphosphonate. Furthermore, the nucleic acid sequences of the loop moieties may comprise polyalkylene glycol at an arbitrary position. Furthermore, the loop moieties may also comprise a peptide.

### 2. Method for producing the circular single-stranded nucleic acid complex

The circular single-stranded nucleic acid complex (dumbbell-shaped nucleic acid complex) of the present invention can be prepared by a production method comprising the following steps:
(1) preparing a linear single-stranded nucleic acid complex comprising a portion of a sense strand, a loop moiety, and a portion of an antisense strand, and a linear single-stranded nucleic acid complex comprising the remaining portion of the sense strand, a loop moiety, and the remaining portion of the antisense strand; and
(2) linking the portion of the sense strand to the remaining portion of the sense strand and the portion of the antisense strand to the remaining portion of the antisense strand.

Alternatively, it can be prepared by a production method comprising the following steps:
(1) preparing sense and antisense strands; and
(2) linking loop moieties to both the ends of double-stranded RNA formed by the sense and antisense strands.

The sense and antisense strands of the dumbbell-shaped nucleic acid complex are designed from the nucleotide sequence of a target gene such that the targeted gene expression can be inhibited. The target gene is generally a gene derived from a eukaryote, preferably from an animal or plant. In the design, inhibition efficiency may be confirmed on a plurality of sense and antisense strands prepared, and, for example, algorithm for siRNA design may be used (references: J.A. Jaeger et al., Methods in Enzymology (1989) 183: 281-306; and D.H. Mathews et al., J. Mol. Biol. (1999) 288: 911-940). For the design, it is preferable that the expression of a gene, other than the target gene, having a sequence similar to the target gene is not inhibited (without off target effect). Moreover, the sequences may be designed such that the 3' end of double-stranded RNA comprising the sense and antisense strands, which is formed from the dumbbell-shaped nucleic acid complex by intracellular or *in-vivo* dicer cleavage, is a protruding end of 1 to 3 U (uracil) bases. The lengths of the sense and antisense strands are not particularly limited and are designed to be lengths of, for example, 19 to 31 nucleotides, preferably 20 to 25 nucleotides, more preferably 20 to 24 nucleotides, even more preferably 20 to 23 nucleotides. Examples of the specific target gene include, but not particularly limited to, overexpressed disease-related genes, viral genes, specifically, the abl/bcr gene for leukemia, the VEGF gene for age-related macular degeneration, and the HCV gene for hepatitis.

The loop moieties are selected from polyalkylene glycol, DNA, DNA-RNA chimera, a derivative thereof, and a combination thereof. The nucleotide sequences of the loop moieties comprise, as shown above, a sequence that does not substantially form a complementary strand.

The preparation of a linear single-stranded nucleic acid complex comprising a portion of a sense strand, a loop moiety, and a portion of an antisense strand, and a linear single-stranded nucleic acid complex comprising the remaining portion of the sense strand, a loop moiety, and the remaining portion of the antisense strand can be performed using a commercially available nucleic acid synthesizer and reagent. The 5' ends of the synthesized linear single-stranded nucleic acid complexes are phosphorylated. When these linear single-stranded nucleic acid complexes are mixed, the sense and antisense strands are partially paired to form a stem with a nick between the portion of the sense strand and the remaining portion of the sense strand and between the portion of the antisense strand and the remaining portion of the antisense strand. This nick is enzymatically or chemically ligated to produce a dumbbell-shaped nucleic acid complex. The enzymatic ligation may utilize ligase, and the ligase is preferably T4 RNA ligase. The chemical ligation may utilize a condensing agent, and the condensing agent is preferably dicyclohexylimide or the like which can generate phosphoric acid ester for the ligation.

Alternatively, sense and antisense strands are respectively synthesized and mixed to form double-stranded RNA. Loop moieties may respectively be linked to both the ends of this double-stranded RNA to produce a dumbbell-shaped nucleic acid complex. When the loop moieties comprise polyalkylene glycol, the 5' end of the nucleic acid is phosphorylated and polyalkylene glycol is linked to the nucleic acid through phosphoric acid ester. Alternatively, both the ends of polyalkylene glycol can be phosphoric acid-esterified and respectively linked to the 3' and 5' ends of the nucleic acid. Alternatively, when the loop moieties are DNA or DNA-RNA chimera, the 5' ends of the synthesized sense and antisense strands and loop moieties are phosphorylated and the loop moieties can respectively be linked by the enzymatic or chemical manner to the ends of the double-stranded RNA.

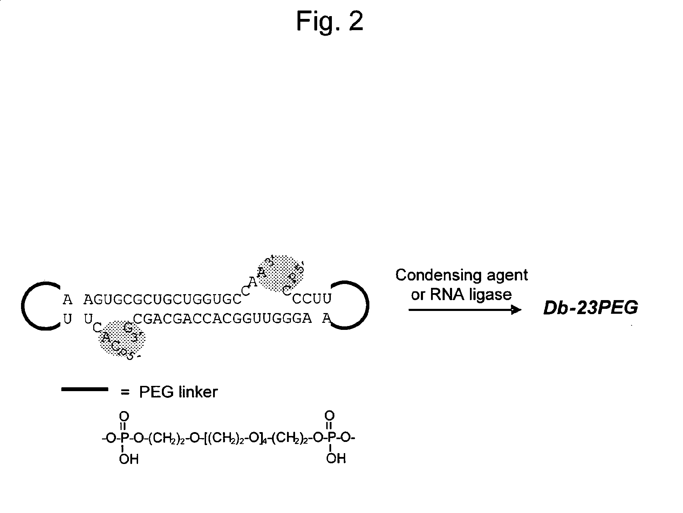

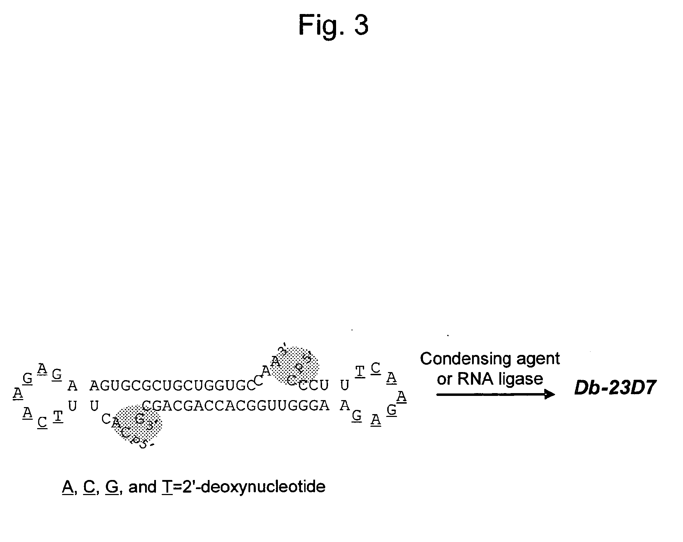

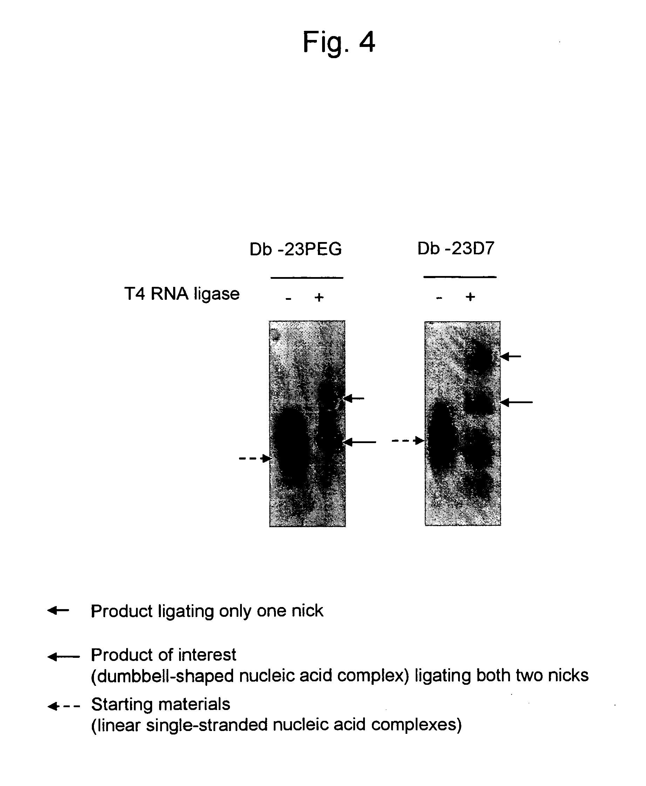

Hereinafter, an example of a method for producing a dumbbell-shaped nucleic acid complex (Db-23PEG; dumbbell-shaped nucleic acid complex comprising a 23-base pair stem and loop moieties comprising polyethylene glycol) will be described specifically with reference to Figure 2. In the diagram, the upper RNA strand in the stem is a sense strand, and the lower RNA strand is an antisense strand.
(1) The RNA strand from an arbitrary nick site of a sense strand RNA sequence to the 5' end thereof is synthesized using a nucleic acid synthesizer based on, for example, the phosphoramidite method, and its 5' end is phosphorylated. Referring to Figure 2, the sequence from the end with the symbol 3' (3'AAC...) of the sense strand to the 5' end (...UGA) thereof is synthesized.
   Next, a loop moiety comprising polyethylene glycol is synthesized. The polyethylene glycol is not particularly limited, and, for example, polyethylene glycol having a molecular weight of approximately 60 to 60000, more preferably approximately 120 to 3000, even more preferably approximately 180 to 1200, particularly preferably approximately 180 to 600 is used. Moreover, a reagent for spacer used in a nucleic acid synthesizer is preferably used. Referring to Figure 2, a loop moiety comprising A (adenine), a polyethylene glycol linker, and U (uracil) is bound to the 5' end of the sense strand.
   Furthermore, the RNA strand from the 3' end of an antisense strand to an arbitrary nick site thereof is synthesized. Referring to Figure 2, the sequence from the 3' end (UCA) of the antisense strand to the end with the symbol 5' thereof is synthesized, and its 5' end is phosphorylated with a phosphorylating reagent. By this step, a linear single-stranded nucleic acid complex can be prepared.
   The numbers of nucleotides in the sense and antisense strands prepared in this step are preferably not the same with each other such that at least one base pairing is formed. The numbers of nucleotides differ by more preferably 1 to 25 nucleotides, even more preferably 5 to 15 nucleotides.
(2) The RNA strand of the remaining portion of the antisense strand sequence synthesized in (1) above is synthesized. Referring to Figure 2, the sequence from the end with the symbol 3' (3'GCG...) of the antisense strand to the 5' end (...GGA) thereof is synthesized.
   Next, a loop moiety comprising polyethylene glycol is synthesized. Referring to Figure 2, a loop moiety comprising A (adenine), a polyethylene glycol linker, and U (uracil) is bound to the 5' end of the antisense strand.
   Furthermore, the RNA strand of the remaining portion of the sense strand is synthesized. Referring to Figure 2, the sequence from the 3' end (UCC) of the antisense strand RNA to the end with the symbol 5' thereof is synthesized, and its 5' end is phosphorylated with a phosphorylating reagent. By this step, a linear single-stranded nucleic acid complex can be prepared.
(3) The linear single-stranded nucleic acid complex prepared in (1) above and the linear single-stranded nucleic acid complex prepared in (2) above are mixed. The sense strand contained in the linear single-stranded nucleic acid complex prepared in (1) above and the antisense strand contained in the linear single-stranded nucleic acid complex prepared in (2) above form a stem by pairing to form a dumbbell-shaped nucleic acid complex having a stem with two nicks, as shown in Figure 2.
   A condensing agent or RNA ligase, for example, T4 RNA ligase, is added thereto to ligate these two nicks. Specifically, the portion of the sense strand in the linear single-stranded nucleic acid complex prepared in (1) above is linked to the remaining portion of the sense strand in the linear single-stranded nucleic acid complex prepared in (2) above, while the portion of the antisense strand in the linear single-stranded nucleic acid complex prepared in (1) above is linked to the remaining portion of the antisense strand in the linear single-stranded nucleic acid complex prepared in (2) above. Referring to Figure 2, A with the symbol 3' of the sense strand is linked to C with the symbol 5' thereof, while C with the symbol 5' of the antisense strand is linked to G with the symbol 3' thereof.
   The ligase reaction conditions preferably involve, for example, incubation at a low temperature or room temperature for approximately 20 hours in a buffer containing polyethylene glycol (PEG), BSA, etc. Addition of the polyethylene glycol added can improve the yield of the dumbbell-shaped nucleic acid complex, and addition of the BSA can prevent inactivation of ligase. Extraction of the dumbbell-shaped nucleic acid complex is not particularly limited, and it is preferable to extract the dumbbell-shaped nucleic acid complex with chloroform and remove redundant polyethylene glycol contained in the reaction solution.
   A dumbbell-shaped nucleic acid complex comprising loop moieties of DNA or DNA-RNA chimera can also be prepared, collected, and purified in the same way as in (1) to (3) above. Figure 3 shows an example of preparation of such a dumbbell-shaped nucleic acid complex (Db-23D7; dumbbell-shaped nucleic acid complex comprising a 23-base pair stem and loop moieties comprising DNA).
   The prepared dumbbell-shaped nucleic acid complex is preferably collected and purified after confirmation of successful preparation of the dumbbell-shaped nucleic acid complex by high-performance liquid chromatography, gel electrophoresis, a test to examine degradation by exonuclease, etc. Figure 4 shows an example of denaturing polyacrylamide gel analysis of the dumbbell-shaped nucleic acid complex thus prepared. In Figure 4, the short arrows represent the band of a product ligating only one nick by T4 RNA ligase, and the long arrows represent the band of the product of interest (dumbbell-shaped nucleic acid complex) ligating both two nicks. The broken-line arrows represent the bands of the linear single-stranded nucleic acid complexes synthesized in (1) and (2) above, used as starting materials for the dumbbell-shaped nucleic acid complex.

### 3. Method for inhibiting gene expression

The dumbbell-shaped nucleic acid complex of the present invention can be introduced into a eukaryotic cell or eukaryote to disable target RNA such that its translation into the protein is inhibited. The cell encompasses animal cells including human cells, plant cells, etc. The dumbbell-shaped nucleic acid complex of the present invention has high *in-vivo* stability (e.g., stability in blood) and can exert effect in a lasting and sustained-release manner. Moreover, it can also be highly safe to living bodies.

For conducing the RNA interference method *in vitro,* the dumbbell-shaped nucleic acid complex can be introduced into a cell by, for example, electroporation, microinjection, lipofection method, or calcium phosphate method.

For conducing the RNA interference method *in vivo,* the dumbbell-shaped nucleic acid complex can be introduced into an animal or plant by, for example, local administration, intravenous administration, or a method using a gene gun.

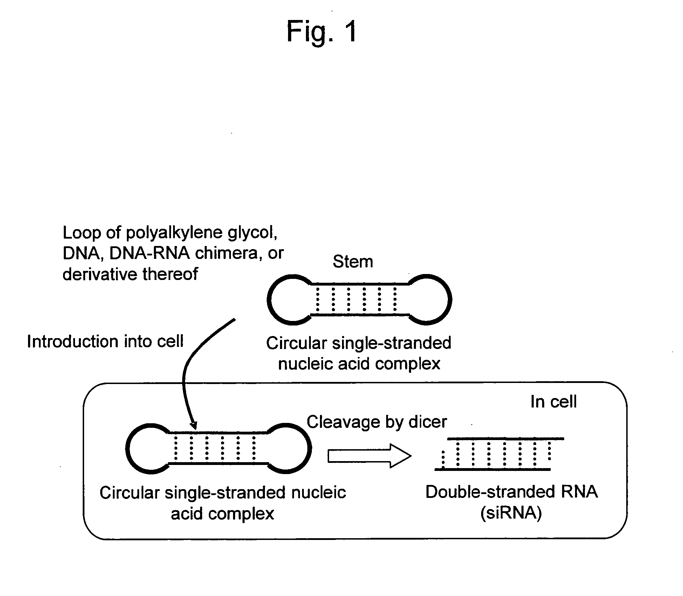

The dumbbell-shaped nucleic acid complex introduced into the cell is cleaved by intracellular dicer to form double-stranded RNA (siRNA) with RNA interference effect in the cell (Figure 1). The siRNA is converted to a single strand, which is integrated into an RNA induced silencing complex (RISC) and then recognizes and degrades target mRNA complementary to the single-stranded RNA, resulting in inhibited expression of the target gene.

### 4. Pharmaceutical composition comprising the circular single-stranded nucleic acid complex

A pharmaceutical composition of the present invention comprises the circular single-stranded nucleic acid complex (dumbbell-shaped nucleic acid complex) as an active ingredient. The dumbbell-shaped nucleic acid complex is formulated in the pharmaceutical composition in an amount of, for example, but not limited to, 0.1% by weight, 0.5% by weight, 1% by weight, 3% by weight, 5% by weight, 10% by weight, 20% by weight, 30% by weight, 40% by weight, 50% by weight, 60% by weight, 70% by weight, 80% by weight, 90% by weight, or 100% by weight, with respect to the total amount of the composition.

Examples of the dosage form of the pharmaceutical composition of the present invention include liquid formulations (solutions, suspensions, emulsions, etc.), solid formulations (lyophilized formulations to be prepared before use, etc.), and liposome (preferably, cationic liposome)-encapsulated formulations. The pharmaceutical composition of the present invention may be administered by systemic or local administration. It has been confirmed recently as to the systemic administration that synthetic siRNA systemically administered to mice and hamsters causes effective silencing of a target gene and in addition, does not influence the amount or activity of endogenous microRNA (miRNA) (John, M. et al. Effective RNAi-mediated gene silencing without interruption of the endogenous microRNA pathway. Nature 449, 745-747 (2007)). The administration route is preferably parenteral administration, and examples thereof include intradermal or hypodermic administration, rectal administration, transmucosal administration, and intravenous administration.

The pharmaceutical composition of the present invention may contain pharmaceutically acceptable excipients or diluents (saline, sterilized water, Ringer's solution, etc.), additives, such as pharmaceutically acceptable buffers, tonicity agents, stabilizers, etc., according to the preparation, dosage form, and so on.

Moreover, the dose of the pharmaceutical composition of the present invention can vary according to the sex, body weight, age, severity, conditions, and so on of a patient.

Examples of the application of the pharmaceutical composition of the present invention include, but not particularly limited to, treatment of cancer, gene disease, viral infection, etc. For example, in the treatment of cancer, the functions of genes or proteins specifically expressed in cancer cells can be inhibited using the pharmaceutical composition of the present invention.

Hereinafter, the present invention will be described more specifically with reference to Examples. However, the present invention is not intended to be limited to these Examples.

### Example 1

### (A) Design of dumbbell-shaped nucleic acid complexes, etc.

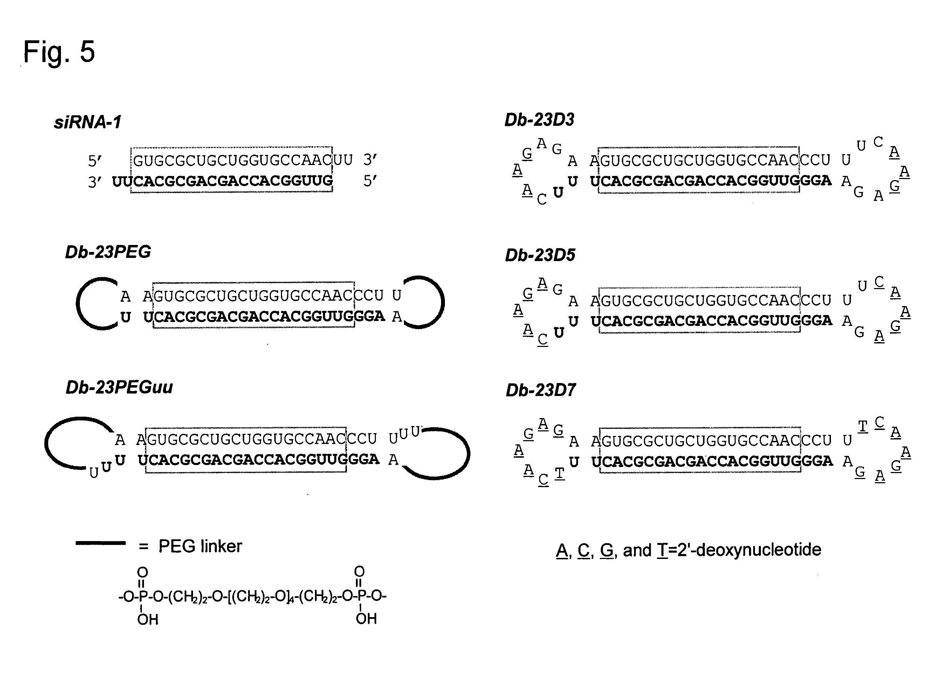

Dumbbell-shaped nucleic acid complexes of the present invention having a 23-base pair stem (SEQ ID NOs: 7 to 13) and double-stranded RNA used as a control for the dumbbell-shaped nucleic acid complexes of the present invention (siRNA, SEQ ID NOs: 5 and 6) were designed (Figure 5). In the diagram, the control siRNA is referred to as siRNA-1. Moreover, in the designation of each designed dumbbell-shaped nucleic acid complex, "Db" means a dumbbell shape; "23" means that the dumbbell-shaped nucleic acid complex has a 23-base pair stem; "PEG" means that polyethylene glycol is contained in the loop moieties. "PEGuu" means that polyethylene glycol is contained in the loop moieties and double-stranded RNA formed by dicer cleavage contains RNA forming a protruding end of UU (two consecutive uracils). "D3", "D5", and "D7" mean that 3, 5, and 7 DNAs, respectively, are contained in the loop moieties. In the stems of the control siRNA-1 and each dumbbell-shaped nucleic acid complex, the upper and lower sequences represent sense and antisense strand sequences, respectively. The sequences indicated in bold type represent a sequence complementary to target RNA. The boxed portions represent a double-stranded RNA moiety common to all of the control siRNA-1 and each dumbbell-shaped nucleic acid complex. A (adenine), C (cytosine), G (guanine), and T (thymine) in the loop moieties of each dumbbell-shaped nucleic acid complex represent 2'-deoxynucleotide. The thick lines shown in the loop moieties of each dumbbell-shaped nucleic acid complex represent a polyethylene glycol (PEG) linker. In this context, the polyethylene glycol is represented by the formula: -[(CH₂)₂-O]ₙ-wherein n is 4. Moreover, for comparison, dumbbell-shaped RNA (Db-23) was designed which consisted of only RNA and had the same sequence as Db-23D3 except that the DNA in the loop moieties was substituted by corresponding RNA.

In the present Example, the dumbbell-shaped nucleic acid complexes, the double-stranded RNA, and the dumbbell-shaped RNA were designed with the sequence of a firefly luciferase expression vector pGL3-Control as a target.

### (B) Preparation of dumbbell-shaped nucleic acid complexes, etc.

### (a) Synthesis of linear single-stranded nucleic acid complexes, etc.

All linear single-stranded nucleic acid complexes used as starting materials for the dumbbell-shaped nucleic acid complexes were synthesized using a nucleic acid synthesizer (Gene World H8-SE) based on the phosphoramidite method. For example, with regard to Db-23PEG and Db-23D7 shown in Figure 5, as shown in Figs. 2 (SEQ ID NOs: 1 and 2) and 3 (SEQ ID NOs: 3 and 4), respectively, a linear single-stranded nucleic acid complex from 3'-terminal RNA at a nick of a sense strand through a loop moiety to a 5'-terminal RNA at a nick of an antisense strand was synthesized using the nucleic acid synthesizer, and its 5' end was phosphorylated. Moreover, a linear single-stranded nucleic acid complex from 3'-terminal RNA at a nick of an antisense strand through a loop moiety to 5'-terminal RNA at a nick of a sense strand was synthesized using the nucleic acid synthesizer, and its 5' end was phosphorylated. Linear single-stranded nucleic acid complexes as starting materials for the other dumbbell-shaped complexes shown in Figure 5 were also synthesized in the same way as above.

Either 2'-O-TBDMS-protected (Proligo) or 2'-O-TOM-protected (Glen Research) form was used as an amidite reagent for RNA synthesis, and Chemical Phosphorylation Reagent (Glen Research) was used in the 5' end phosphorylation. Moreover, Spacer Phosphoramidite 18 (Glen Research) was used as PEG in the loop moieties. RNA was deprotected according to a standard method, and the full-length product was purified on a denaturing polyacrylamide gel.

Linear single-stranded RNAs used as starting materials for the control siRNA-1 were synthesized using said nucleic acid synthesizer, followed by purified. With regard to linear single-stranded RNAs used as starting materials for Db-23 for comparison, linear single-stranded RNA in which 4- or 5-nucleotide portions in the loop moieties were respectively linked to both the ends of the sense strand, and linear single-stranded RNA in which the remaining 5- or 4-nucleotide portions of the loop moieties were respectively linked to both the ends of the antisense strand were synthesized using the nucleic acid synthesizer, followed by purification.

### (b) Preparation of dumbbell-shaped nucleic acid complexes through ligation reaction using T4 RNA ligase

A reaction solution containing T4 RNA ligase was formulated to have the following final composition:
µM each linear single-stranded nucleic acid complex
0.2 to 0.4 units/µL T4 RNA ligase
0.006% BSA
25% PEG6000
50 mM Tris-HCl (pH 7.5)
10 mM MgCl₂
10 mM DTT
1 mM ATP
(reaction scale: 2.5 mL)

Specifically, the two 5'-phosphorylated linear single-stranded nucleic acid complexes synthesized and purified in (a) above were dissolved at the combined concentration of 4 µM in 100 mM Tris-HCl (pH 7.5), 20 mM MgCl₂, 20 mM DTT, and 2 mM ATP buffer (2x buffer, half the amount of the final reaction solution), and the mixture was heated at 90°C for 3 minutes and then allowed to cool slowly to room temperature. Then, an aqueous BSA solution, an aqueous PEG6000 solution, and T4 RNA ligase were added according to the composition of the reaction solution shown above, and the mixture was incubated at room temperature for approximately 20 hours. The reaction solution (2.5 mL) was extracted two times with chloroform (2.5 mL) (removal of PEG6000). RNA was collected by alcohol precipitation, and circular forms were isolated using denaturing PAGE (10% PAGE, 7 M urea, 25% formamide, 1x TBE, 1 mm thick). The electrophoretic profiles are shown in Figure 4. In Figure 4, the bands represented by the long arrows are circular forms (dumbbell-shaped nucleic acid complexes). Here, the yield of each dumbbell-shaped nucleic acid complex was approximately 50% for both Db-23PEG and Db-23D7 with respect to the amount (100%) of the two linear single-stranded nucleic acid complexes used as starting materials for each dumbbell-shaped nucleic acid complex. The bands containing the product of interest were visualized by the UV shadowing method, then excised, and finely pulverized, followed by three extractions with 4 mL of elution buffer (0.2 M NaCl, 10 mM EDTA (pH 8.0)). The extracts were desalted (eluted with 6 mL of 50% aqueous acetonitrile solution) using a Sep-Pak cartridge, concentrated using a centrifuge evaporator, and further alcohol-precipitated in the presence of sodium acetate. The obtained nucleic acid complexes were dissolved in ultrapure water and appropriately diluted. Then, UV absorption spectra were measured to quantify solution concentrations. The yield was approximately 10 to 25% for Db-23PEG and Db-23D7 with respect to the amount (100%) of the two linear single-stranded nucleic acid complexes used as starting materials for each dumbbell-shaped nucleic acid complex. The other dumbbell-shaped nucleic acid complexes had almost the same yields thereas.

### (c) Preparation of siRNA and Db-23

The control siRNA-1 was prepared by mixing and annealing the sense and antisense strands prepared in (a) above. siRNA-1 was collected by ethanol precipitation and purified on PAGE.

Db-23 for comparison was prepared by mixing and annealing the linear single-stranded RNA comprising the sense strand and the linear single-stranded RNA comprising the antisense strand, prepared in (a) above, and respectively linking loop moieties thereto using T4 RNA ligase. The reaction conditions of T4 RNA ligase were the same as above except that: the reaction scale of 2.5 mL was changed to 25 µL; the T4 RNA ligase concentration of 0.2 to 0.4 units/µL was changed to 2.0 units/µL; the heating at 90°C for 3 minutes was changed to heating at 65°C for 5 minutes; and the incubation at room temperature for approximately 20 hours was changed to incubation at 11°C for 20 hours. Db-23 was collected by ethanol precipitation and purified on PAGE.

### (C) Test on biological stability of dumbbell-shaped nucleic acid complexes in serum

5 µM each dumbbell-shaped nucleic acid complex, control siRNA-1, or Db-23 for comparison was heated at 90°C for 3 minutes in 1x annealing buffer (100 mM potassium acetate, 30 mM HEPES-KOH (pH 7.4), 2 mM magnesium acetate) and then allowed to cool slowly. 4 µL of this nucleic acid solution was mixed with 16 µL of bovine serum (GIBCO) and incubated at 37°C. After 2, 4, 6, and 8 hours, a portion of the reaction solution was sampled and analyzed on 15% non-denaturing PAGE. The gel was stained with SYBR Green I to visualize bands for quantification.

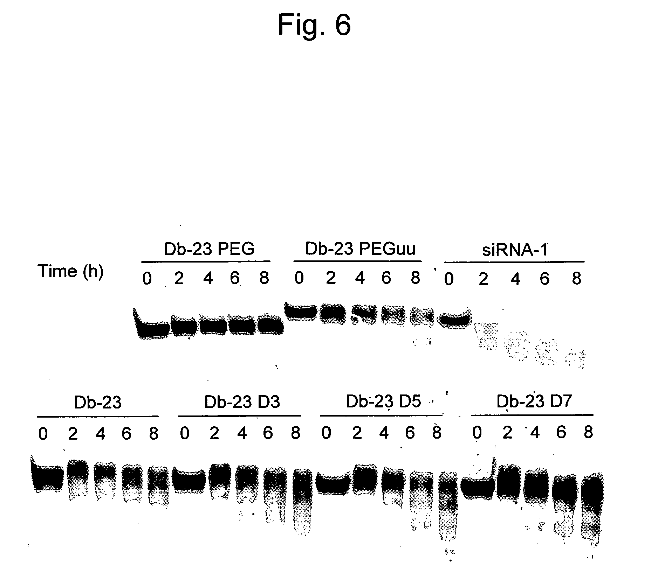

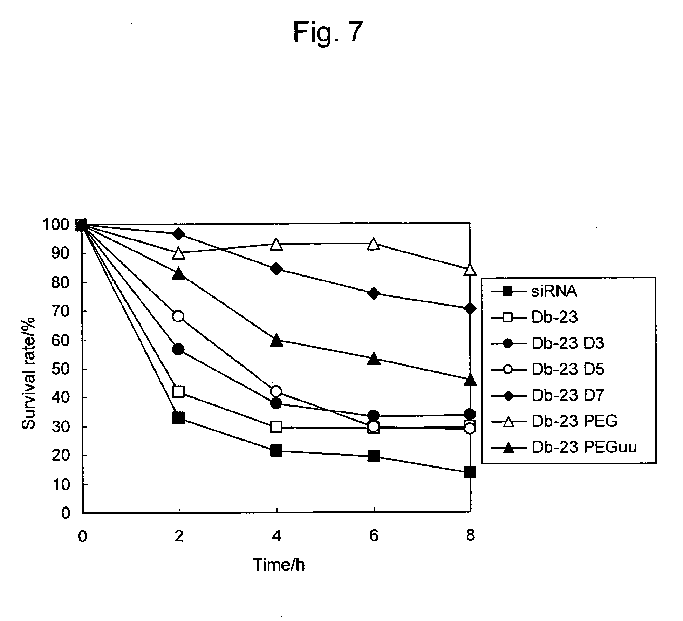

Time-dependent change in the quantification results based on bands showed that the control siRNA-1 and Db-23 for comparison were decreased to approximately 30% and approximately 40%, respectively, after 2 hours, whereas all the dumbbell-shaped nucleic acid complexes of the present invention were stable (Figs. 6 and 7). Particularly, 70% or more of Db-23PEG and Db-23D7 remained even after 8 hours or later, demonstrating their high stability in blood.

### (D) Test on biological stability of dumbbell-shaped nucleic acid complexes in cell extracts

HL60 cells (RIKEN CELL BANK) were cultured, and cell extracts were prepared. The cell extracts were mixed at a final protein concentration of 10 mg/mL with 2.5 µM each dumbbell-shaped nucleic acid complex, control siRNA-1, or Db-23 for comparison and incubated at 37°C. After 0.5, 1, 2, and 4 hours, a portion of the reaction solution was sampled. This sample was analyzed on 15% non-denaturing PAGE. The gel was stained with SYBR Green I to visualize bands for quantification.

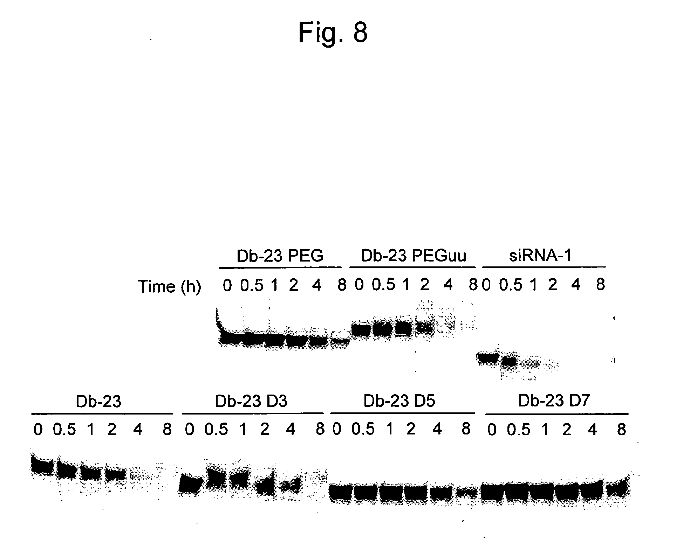

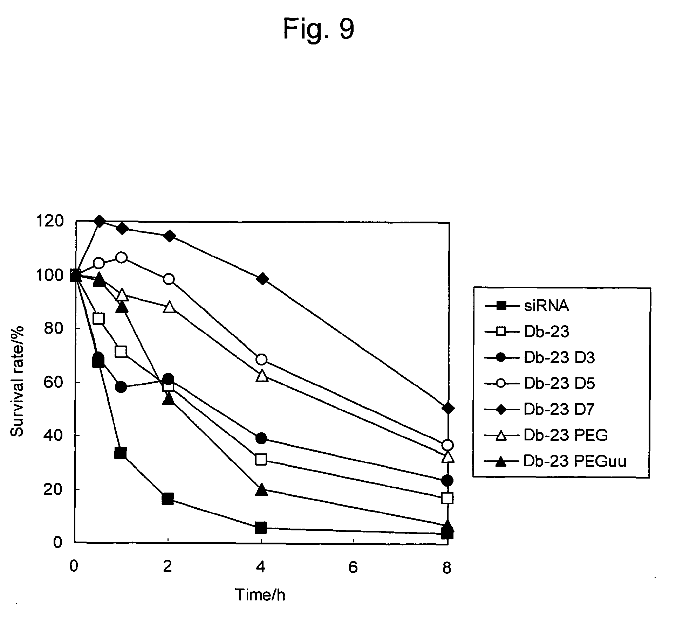

Time-dependent change in the quantification results based on bands showed that the control siRNA-1 was already degraded to 40% or lower in 1 hour, whereas the dumbbell-shaped nucleic acid complexes of the present invention were exceedingly stable with Db-23D7 most stable (Figs. 8 and 9). Db-23D7 remained with little degradation even after 4 hours.

### (E) Measurement of RNA interference effects of dumbbell-shaped nucleic acid complexes using cultured mammalian cell system

The RNA interference effects of the dumbbell-shaped nucleic acid complexes in cultured mammalian cells were measured using Dual-luciferase Reporter Assay System (Promega).

NIH 3T3 cells (RIKEN CELL BANK) were cultured at 37°C in a 5% CO₂ atmosphere in a Dulbecco's modified eagle's medium (DMEM, GIBCO) containing 10% fetal calf serum (FCS, Invitrogen/GIBCO). The culture solution was dispensed (100 µL; 1.6x10⁴ cells/well) to a 96-well plate. The cells were further cultured at 37°C for 24 hours in a 5% CO₂ atmosphere. The cells in approximately 70% confluent state were cotransfected with two plasmid vectors (pGL3-Control and pRL-TK (internal standard), both Promega) and each dumbbell-shaped nucleic acid complex or control siRNA-1 with a transfection reagent Gene Silencer (Genlantis) according to the protocol included in the transfection reagent. The concentration conditions for the transfection involved 0.2 µg pGL3-Control/0.02 µg pRL-TK/25 nM each dumbbell-shaped nucleic acid complex or control siRNA-1 (final amount: 100 µL).

Specifically, 55.6 µL of serum-free DMEM medium was added to the cells, and in this state, a mixed solution for transfection having the following composition was added to perform cotransfection:

| | |
|---|---|
| Gene Silencer | 1 µL |
| DMEM medium | 25 µL |
| siRNA dilution | 2.5 µL |
| DMEM medium | 15 µL |
| RNA (5 pmol/µl) | 0.5 µl |
| pGL3-Control (1 µg/µl) | 0.2 µL |
| pRL-TK (0.1 µg/µl) | 0.2 µL |
| | 44.4 µL |

After the transfection, the reaction solution was incubated at 37°C for 4 hours in a 5% CO₂ atmosphere, and 100 µL of DMEM medium containing 20% serum was added to each well. After further incubation at 37°C for 20 hours, the expression level of luciferase was quantified using Dual-luciferase Reporter Assay System (Promega) according to the protocol included therein (conditions; the amount of reagent: 30 µL, delay time: 2 seconds, read time: 10 seconds, apparatus: Wallac ARVO SX 1420 Multilabel Counter). For comparison, conditions involving only a buffer (control) were also evaluated in the same way as above. The luminescence intensity of firefly luciferase was corrected based on the luminescence intensity of *Renilla* luciferase as an internal standard.

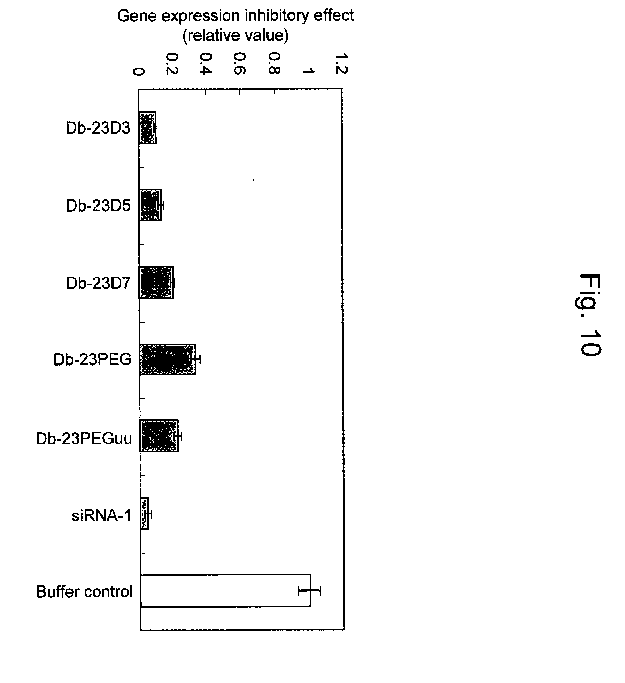

The results are shown in Figure 10. siRNA-1 and Db-23D3 were demonstrated to have almost equivalent activity. Furthermore, Db-23D7, Db-23PEG, etc. were observed to have sufficient inhibitory activity, albeit smaller than siRNA.

These Examples demonstrated that the dumbbell-shaped nucleic acid complexes have sufficient stability in serum and in cell extracts and exert sufficient RNA interference effect in mammalian cells.

### Industrial Applicability

A circular single-stranded nucleic acid complex of the present invention, when administered intracellularly or *in vivo*, is specifically recognized by dicer and cleaved at loop moieties on both sides to form double-stranded RNA (Figure 1). This double-stranded RNA can have RNA interference effect. The circular single-stranded nucleic acid complex of the present invention has significantly excellent effects that it has more excellent *in-vivo* stability (e.g., stability in blood) than conventional double-stranded RNA and can exert lasting and sustained-release effect in RNA interference. Thus, a drug containing the circular single-stranded nucleic acid complex that has lasting and sustained-release effect and can impart RNA interference effect can be provided. This drug can be administered systemically or directly to the affected area and can be administered at a decreased dose.

Furthermore, the circular single-stranded nucleic acid complex having high *in-vivo* stability can be produced simply and efficiently by a production method of the present invention.

The dumbbell-shaped nucleic acid complex of the present invention is expected to have wide application in, for example, the medical or agricultural field. The dumbbell-shaped nucleic acid complex of the present invention can be used for various purposes, for example, drug development, agricultural chemical development, and elucidation of the functions of particular genes or proteins in plants or animals or their cells, for example, elucidation of the functions by the knockout method.

All publications, patents, and patent applications cited herein are incorporated herein by reference in their entirety.

## Claims

1. A circular single-stranded nucleic acid complex comprising a sense strand RNA sequence, an antisense strand RNA sequence complementary to the sense strand RNA sequence, and two identical or different loop moieties connecting the sense and antisense strands, wherein the sense and antisense strands form a stem by pairing, and the loop moieties are selected from the group consisting of polyalkylene glycol, DNA, DNA-RNA chimera, a derivative thereof, and a combination thereof.

2. The circular single-stranded nucleic acid complex according to claim 1, wherein the circular single-stranded nucleic acid complex is intracellularly converted to double-stranded RNA having RNA interference effect.

3. The circular single-stranded nucleic acid complex according to claim 1 or 2, wherein the RNA sequence forming the stem has 80 to 100% identity to a target RNA sequence.

4. The circular single-stranded nucleic acid complex according to any one of claims 1 to 3, wherein the polyalkylene glycol is polyethylene glycol.

5. The circular single-stranded nucleic acid complex according to any one of claims 1 to 4, wherein the stem has a length of 19 to 31 nucleotides.

6. A method for producing the circular single-stranded nucleic acid complex according to any one of claims 1 to 5, comprising the following steps:
(1) preparing a linear single-stranded nucleic acid complex comprising a portion of a sense strand, a loop moiety, and a portion of an antisense strand, and a linear single-stranded nucleic acid complex comprising the remaining portion of the sense strand, a loop moiety, and the remaining portion of the antisense strand; and
(2) linking the portion of the sense strand to the remaining portion of the sense strand and the portion of the antisense strand to the remaining portion of the antisense strand.

7. A method for producing the circular single-stranded nucleic acid complex according to any one of claims 1 to 5, comprising the following steps:
(1) preparing sense and antisense strands; and
(2) linking loop moieties to both the ends of double-stranded RNA formed by the sense and antisense strands.

8. A method for inhibiting *in vitro* the expression of a gene encoding a protein, comprising introducing the circular single-stranded nucleic acid complex according to any one of claims 1 to 5 into a eukaryotic cell to disable target RNA such that its translation into the protein is inhibited.

9. A method for inhibiting the expression of a gene encoding a protein, comprising introducing the circular single-stranded nucleic acid complex according to any one of claims 1 to 5 into a non-human animal, a plant, or a cell thereof to disable target RNA such that its translation into the protein is inhibited.

10. A pharmaceutical composition comprising the circular single-stranded nucleic acid complex according to any one of claims 1 to 5 as an active ingredient.


SEQUENCE LISTING

<110>  RIKEN

<120>  Circular single-stranded nucleic acid complex and method of
       producing the same

<130>  PH-3865-PCT

<150>  JP 2008-035310
<151>  2008-02-15

<160>  17

<170>  PatentIn version 3.4

<210>  1
<211>  20
<212>  RNA
<213>  Artificial

<220>
<223>  synthetic

<400>  1
aagugcgcug cuggugccaa                                                  20

<210>  2
<211>  20
<212>  RNA
<213>  Artificial

<220>
<223>  synthetic

<400>  2
aaggguuggc accagcagcg                                                  20

<210>  3
<211>  32

<212>  DNA
<213>  Artificial

<220>
<223>  synthetic DNA/RNA binding molecule

<220>
<222>  1..5
<223>  RNA

<220>
<222>  13..32
<223>  RNA

<400>  3
cacuutcaag agaagugcgc ugcuggugcc aa                                    32

<210>  4
<211>  32
<212>  DNA
<213>  Artificial

<220>
<223>  synthetic DNA/RNA binding molecule

<220>
<222>  1..5
<223>  RNA

<220>
<222>  13..32
<223>  RNA

<400>  4
cccuutcaag agaaggguug gcaccagcag cg                                    32

<210>  5
<211>  21
<212>  RNA

<213>  Artificial

<220>
<223>  synthetic

<400>  5
gugcgcugcu ggugccaacu u                                                21

<210>  6
<211>  21
<212>  RNA
<213>  Artificial

<220>
<223>  synthetic

<400>  6
guuggcacca gcagcgcacu u                                                21

<210>  7
<211>  25
<212>  RNA
<213>  Artificial

<220>
<223>  synthetic

<400>  7
aagugcgcug cuggugccaa cccuu                                            25

<210>  8
<211>  25
<212>  RNA
<213>  Artificial

<220>
<223>  synthetic

<400>  8

aaggguuggc accagcagcg cacuu                                    25

<210> 9
<211> 27
<212> RNA
<213> Artificial

<220>
<223> synthetic

<400> 9
aagugcgcug cuggugccaa cccuuuu                                  27

<210> 10
<211> 27
<212> RNA
<213> Artificial

<220>
<223> synthetic

<400> 10
aaggguuggc accagcagcg cacuuuu                                  27

<210> 11
<211> 64
<212> DNA
<213> Artificial

<220>
<223> synthetic circular DNA/RNA binding molecule

<220>
<222> 1..26
<223> RNA

<220>
<222> 30..58
<223> RNA

<220>
<222>  62..64
<223>  RNA

<400>  11
agugcgcugc uggugccaac ccuuucaaga gaaggguugg caccagcagc gcacuuucaa      60

gaga                                                                    64



<210>  12
<211>  64
<212>  DNA
<213>  Artificial

<220>
<223>  synthetic circular DNA/RNA biding molecule

<220>
<222>  1..25
<223>  RNA

<220>
<222>  31..57
<223>  RNA

<220>
<222>  63..64
<223>  RNA

<400>  12
agugcgcugc uggugccaac ccuuucaaga gaaggguugg caccagcagc gcacuuucaa      60

gaga                                                                    64



<210>  13
<211>  64
<212>  DNA
<213>  Artificial

<220>
<223> synthetic circular DNA/RNA binding molecule

<220>
<222> 1..24
<223> RNA

<220>
<222> 32..56
<223> RNA

<220>
<222> 64
<223> RNA

<400> 13
agugcgcugc uggugccaac ccuutcaaga gaaggguugg caccagcagc gcacuutcaa 60

gaga 64

<210> 14
<211> 11
<212> DNA
<213> Artificial

<220>
<223> synthetic

<400> 14
gattagtcac t 11

<210> 15
<211> 9
<212> DNA
<213> Artificial

<220>
<223> synthetic

<400> 15

agagaactt                                                                9

<210>  16
<211>  9
<212>  DNA
<213>  Artificial

<220>
<223>  synthetic

<400>  16
ttcaagaga                                                                9

<210>  17
<211>  9
<212>  DNA
<213>  Artificial

<220>
<223>  synthetic

<400>  17
tgtgctgtc                                                                9